(19)

**Europäisches Patentamt**
**European Patent Office**
**Office européen des brevets**

(11) **EP 3 656 296 A1**

(12) **EUROPEAN PATENT APPLICATION**
published in accordance with Art. 153(4) EPC

(43) Date of publication:
**27.05.2020 Bulletin 2020/22**

(21) Application number: **18834509.4**

(22) Date of filing: **30.04.2018**

(51) Int Cl.:
*A61B 5/03* (2006.01)          *A61B 5/0476* (2006.01)
*G16H 10/60* (2018.01)          *G16H 50/20* (2018.01)

(86) International application number:
**PCT/ES2018/070334**

(87) International publication number:
**WO 2019/016420 (24.01.2019 Gazette 2019/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.07.2017 ES 201730943**

(71) Applicant: **Fundación para la Investigación
Biomédica del
Hospital Universitario La Princesa
28006 Madrid (ES)**

(72) Inventors:
• **SANZ GARCIA, Ancor
28006 Madrid (ES)**

• **PASTOR GOMEZ, Jesús
28006 Madrid (ES)**
• **VEGA ZELAYA, Lorena Carolina
28006 Madrid (ES)**
• **TORRECILLA LOPEZ, María del Carmen
28006 Madrid (ES)**
• **VEGA GONZALEZ, María Gema
28006 Madrid (ES)**
• **MONASTERIO CHICHARRO, Fernando
28006 Madrid (ES)**
• **GARCIA DE SOLA, Rafael
28006 Madrid (ES)**
• **PULIDO RIVAS, Paloma
28010 Madrid (ES)**
• **TORRES DIAS, Cristina Virginia
28010 Madrid (ES)**

(74) Representative: **Temiño Ceniceros, Ignacio
Calle Amador de los Rios 1-1°
28010 Madrid (ES)**

(54) **NON-INVASIVE METHOD FOR DETERMINING INTRACRANIAL PRESSURE USING THE BIOELECTRICAL ACTIVITY OF THE BRAIN**

(57)    The present invention relates to a non-invasive method for determining changes in intracranial pressure using data obtained from an electroencephalogram recorded for a patient comprising:
a) determining the values of the spectral analysis and network variables for the electroencephalogram,
b) determining the endogenous variable X for a transfer function the exogenous variables of which are the values for the variables obtained in step a),
whereby changes in the endogenous variable X are indicative of changes in the value of the intracranial pressure; the present invention also relates to a device for carrying out the method of the present invention.

EP 3 656 296 A1

**Description**

**Field of the invention**

The present invention falls within the general field of biomedicine, and in particular relates to a non-invasive method and system for determining changes in intracranial pressure using data obtained from an electroencephalogram recorded for a patient.

**State of the art**

Continuous multimodal monitoring of neurocritical patients is a widespread practice nowadays in most intensive care units (ICUs). Both neurosurgeons and intensive care specialists have a large arsenal of techniques that enable them to continuously monitor critical variables in patients with a wide variety of pathologies. Cases involving traumatic brain injury (TBI) and subarachnoid haemorrhage (SAH) are of particular interest. In these patients, it is useful to monitor intracranial pressure (ICP), cerebral perfusion pressure (CPP), tissue oxygen pressure (PtiO2) and electrical activity of the brain by using electroencephalography (EEG), among many other variables. The value of using it to identify, prevent and treat secondary injuries which can worsen the primary pathology of the patient is certainly indisputable nowadays [Le Roux, P., Menon, D. K., Citerio, G., Vespa, P., Bader, M. K., Brophy, G. M., ...& Badjatia, N. (2014). Consensus summary statement of the international multidisciplinary consensus conference on multimodality monitoring in neurocritical care. Neurocritical care, 21(2), 1-26]. Multimodal monitoring is helpful in those cases in which the clinical examination of the patient may be impossible due to the effects of sedoanalgesia or in those cases in which the patient is in an altered state of consciousness, such as when they are in a coma. Moreover, the information provided by the monitoring equipment usually precedes the clinical information, for which reason changes in the monitored variables help detect changes in the underlying physiological processes and therefore predict any change in the clinical condition.

One of the variables that must be monitored in any neurocritical patient is the ICP, the measurement of which is highly invasive since it is carried out by means of a sensor which measures the pressure of the cerebrospinal fluid either intraparenchymally or intraventricularly, and therefore requires surgery for the placement thereof.

Therefore, there is a need to provide a non-invasive alternative in order to determine the risk associated with an increase in intracranial pressure via a non-invasive method.

**Description of the invention**

The present invention solves the problems described in the state of the art since it refers to a non-invasive method and system for determining intracranial pressure in a subject. Thus, in a first aspect, the present invention relates to a non-invasive method for determining changes in intracranial pressure using data from an electroencephalogram recorded for a patient (hereinafter, method of the present invention) comprising:

> a) determining the values of the spectral analysis and network variables for the electroencephalogram,
> b) determining the endogenous variable X for a transfer function the exogenous variables of which are the values for the variables obtained in step a),

whereby changes in the endogenous variable X are indicative of changes in the value of the intracranial pressure.

In a particular embodiment of the present invention, the spectral analysis variables for the electroencephalogram determined in step a) are selected from: bands lower than delta (<1Hz), delta (1-4Hz), theta (4-7Hz), alpha (8-12Hz), beta (12-30Hz), gamma (30-100Hz), bands greater than gamma (>100Hz), spectral entropy of all frequencies. In a more particular embodiment, at least two of the spectral analysis variables are determined in step a) of the method of the present invention.

In one particular embodiment of the present invention, the network analysis variables for the electroencephalogram determined in step a) are selected from among the variables obtained from the synchronisation between the different electrodes of the electroencephalogram: density of links, average clustering coefficient, mean path length and degree of each electrode of the encephalogram or node. In a more particular embodiment, at least two of the network analysis variables are determined in step a) of the method of the present invention.

In the present invention, channel and node refer to the same element, namely the data obtained from an encephalogram, but from different perspectives. Therefore, channel refers to the signal received through each electrode of the electroencephalogram. When the network analysis is performed, instead of a channel it is called a node and it refers to each of the points making up the network.

In a more particular embodiment of the present invention, the value of the spectral analysis and network variables of step a) is determined by means of the following steps:

I. calculating the power spectrum in the neurophysiological records from each electrode or channel, by means of the Fourier transform;

II. calculating the relative power of each of the characteristic frequency bands of the electroencephalogram;

III. calculating the value of entropy and spectral entropy;

IV. calculating synchronisation measurements between all pairs of neurophysiological records using Pearson correlation, Mutual Information and Phase Synchronisation;

V. calculating the value of the density of links, average clustering coefficient, mean path length and degree of each node;

VI. estimating all the previous measurements in consecutive time windows.

In a particular embodiment, the method of the present invention optionally comprises determining variables associated with the heart rate variability of an electrocardiogram recorded for a patient.

In another particular embodiment, the method of the present invention optionally comprises determining the variables associated with the sedation of a patient.

In a second aspect, the present invention relates to a device for determining intracranial pressure in a patient (hereinafter, device of the present invention) by means of the method of the present invention, which comprises means for determining the values of the spectral analysis and network variables for an electroencephalogram; and means for calculating the transfer function from said variables.

More particularly, the device of the present invention further comprises a microprocessor.

More particularly, the device of the present invention comprises means for recording the electroencephalographic and electrocardiographic signals.

More particularly, the device of the present invention comprises a supervised learning algorithm.

### Description of the figures

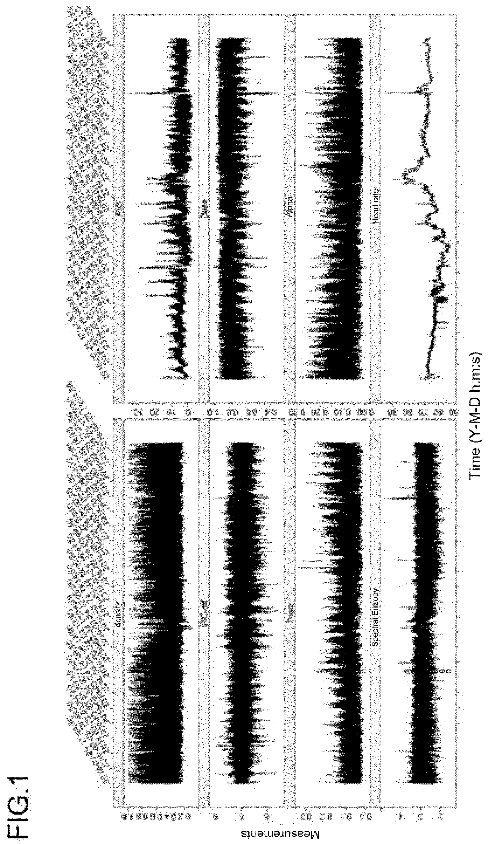

Figure 1 shows the continuous monitoring of 3 consecutive days of different measurements of the EEG, ICP and heart rate, in each of the 5-second time windows. The measurements derived from the EEG are: the density of links, "density", the relative spectral power in the Delta, Theta, Alpha bands and spectral entropy. The measurements of the ICP and the differential thereof, "diff-ICP", can also be observed, as well as the heart rate, estimated by means of an electrode responsible for measuring cardiac activity (V3 lead).

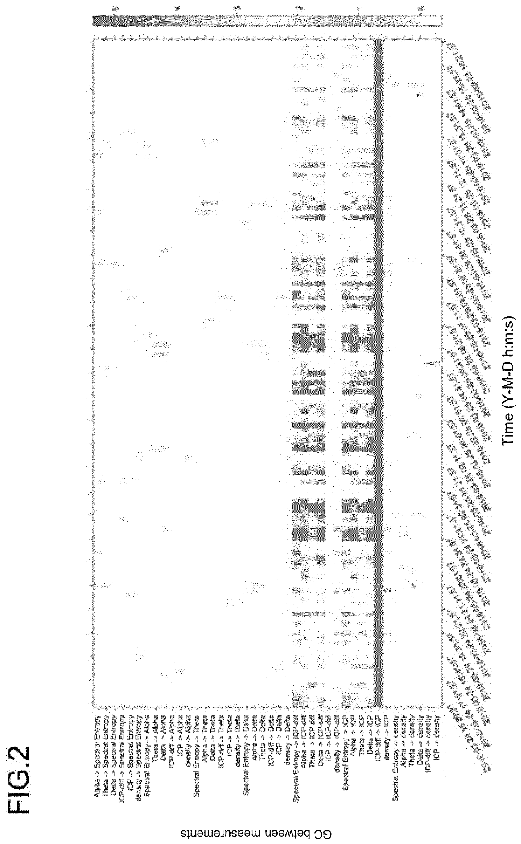

Figure 2 shows estimates of the Granger causality (GC) between all the variables throughout one day of continuous monitoring. When the value of F, determined by Equation (2), for this pair of variables is not significant ($P<0.05$), a zero was put in that time window. Otherwise, the corresponding value has been graphed. The colour scale of these values is found to the right of the figure, the highest values being those in red. In order to make the graph clearer, all the F values greater than 5 have been made equal to 5. Since the non-significant values of F have been zeroed, they are blank in Figure 2.

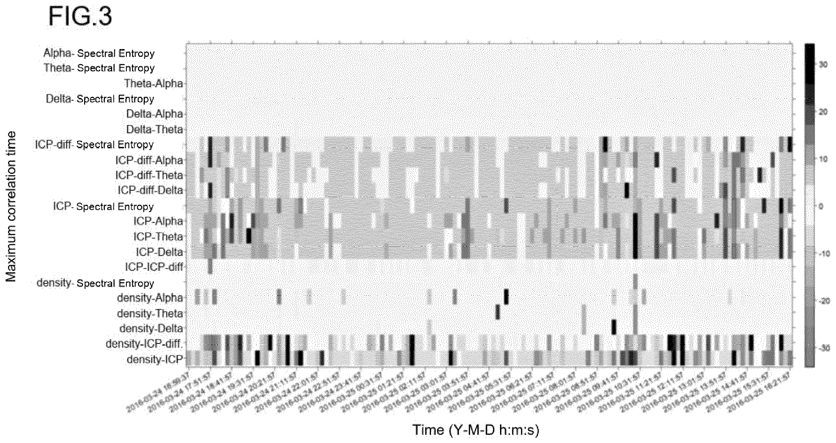

Figure 3 shows the lag in cross-correlation between the different variables. It can be observed that there is no correlation with lag (or lead) between variables derived from the EEG, but it does exist between the variables from the EEG and the ICP (and the differential thereof). In most cases, it is clear that there is lead (coloured blue) in the maximum value of the correlation, this fact implying that the variables derived from the EEG have lag with respect to the ICP. According to the colour scale of the graph, on average this lag is between 5 and 10 steps. Given that these steps are actually 5-second time windows, this would correspond to a lag of 25 and 50 seconds.

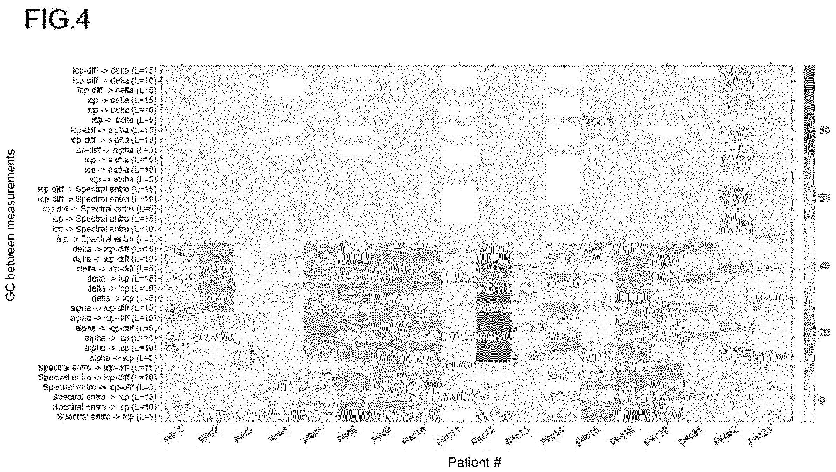

Figure 4 shows the percentages of Granger causality (GC) for the patient sample.

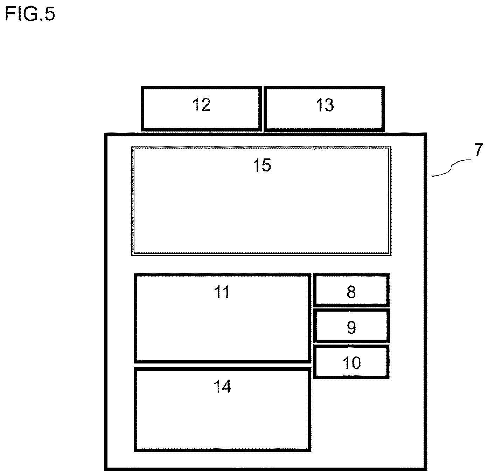

Figure 5 shows a particular embodiment of the device of the present invention (7), comprising means for determining the values of the spectral analysis (8) and network (9) variables for an electroencephalogram; and means for calculating the transfer function (10) from said variables, and as optional components: a microprocessor (11), means for recording the electroencephalographic signal (12) and the electrocardiographic signal (13), a supervised learning algorithm (14) and a screen for seeing the data (15).

### Detailed description of the invention

We have analysed the records of 18 patients (8 of whom are women) admitted to the ICU of the Hospital de la Princesa during the period from October 2015 to March 2017. All patients were monitored continuously with scalp EEG and ICP. The data analysis was performed retrospectively and always with the informed consent of the patients or their families. The research study was approved by the Ethics Committee of the Hospital de la Princesa. Inclusion criteria were as follows: patients of both sexes, over 18 years of age, having TBI or SAH, Glasgow scale less than 9, ICP monitoring. The exclusion criteria included: patients with a stay of less than one week, impossibility of continuous recording of EEG. The continuous monitoring of EEG was done by means of 19 scalp electrodes, mounted in a standard 10-20 electrode placement configuration. The records have been sampled at a frequency of 500 Hz and a monopolar assembly referencing the midline of the electrodes has always been used, meaning, (Fz + Cz + Pz)/3. The records were acquired continuously for a period (on average) of 5.2±2.3 days for each patient. In order to eliminate segments of records with artefacts from patient manipulation, interference with other equipment, etc., a video camera was installed which enables the patient to be monitored continuously.

Each EEG record has been divided into 5-second windows which, due to the fact that they are sampled at 500 Hz, correspond to windows with 2500 data points in each of the 19 channels. We have verified that in these 5-second windows the records have an acceptable weak stationarity and therefore we have calculated several measurements, both spectral and network ones. In particular, we have calculated the relative spectral power, meaning, the power in each band with respect to the total power, for each of the Delta, Theta, Alpha, Beta and Gamma bands, as well as the density of links that the interaction network has [Sanz-García, A., Vega-Zelaya, L., Pastor, J., Sola, R. G., & Ortega, G. J. (2017). Towards Operational Definition of Postictal Stage: Spectral Entropy as a Marker of Seizure Ending. Entropy, 19(2), 81.]. For the purpose of calculating this last measurement, first we have calculated an estimator for the level of synchronisation existing between the activity recorded between each pair of electrodes. We have used the following statistics as synchronisation estimators: Pearson correlation, phase synchronisation, mutual information and coherence [Mezeiova K., Paluu M. (2012). Comparison of coherence and phase synchronization of the human sleep electroencephalogram. Clin Neurophysiol 123, 1821-1830]. We have obtained estimates of all these measurements at 5-second intervals, meaning, twelve values per minute, in non-overlapping windows.

Moreover, the ICP has also been continuously recorded [Kirkman, M. A., & Smith, M. (2013). Intracranial pressure monitoring, cerebral perfusion pressure estimation, and ICP/CPP-guided therapy: a standard of care or optional extra after brain injury?. British journal of anaesthesia, aet418; Kristiansson, H., Nissborg, E., Bartek, J., Andresen, M., Reinstrup, P., & Romner, B. (2013) Measuring Elevated Intracranial Pressure through Noninvasive Methods: A Review of the Literature. J Neurosurg Anesthesiol, 25 (4):372-85.] in those patients in whom, due to their pathology and particular condition, it has been indicated by neurosurgeons and intensive care specialists. In these cases, we have obtained continuous intracranial pressure records, measured by means of a Camino fibreoptic sensor [Martínez-Mañas, R. M., Santamarta, D., de Campos, J. M., & Ferrer, E. (2000). Camino® intracranial pressure monitor: prospective study of accuracy and complications. Journal of Neurology, Neurosurgery & Psychiatry, 69(1), 82-86.] which, by means of a transducer that is located at the tip of an optical fibre and inserted into the parenchyma, allowing intracranial pressure values to be obtained thanks to changes in the intensity of light reflected in a mirror which is moved by the ICP. The ICP data was obtained and stored by means of a programme specifically designed for this purpose, NeuroPic. The sampling time of the ICP is approximately 2.9 seconds between successive values, although this can vary. In order to remove this variation in the sampling frequency and obtain "average" ICP data at times coinciding with those of the EEG time windows, we have re-sampled this signal with a sampling time of 5 seconds between consecutive data points. In this manner, we have ICP values for every minute, at 0 seconds, 5 seconds, 10 seconds, etc. until 12 ICP values are obtained for every minute. This temporal discretisation coincides with the one performed for the values of the time windows for the measurements calculated from the EEG records, as explained in the previous paragraph. It is worth mentioning that the ICP sensor tends to have drift in the initial calibration thereof, meaning that the zero of the initial pressure, calibrated with respect to the atmospheric pressure just before being inserted into the parenchyma, appears with values other than zero when taken off. In order to correct this deviation, we have removed the slope calculated between the initial zero and the final deviation value from the time series of the ICP. Generally, this value does not exceed ± 2 mmHg.

We have also paired up the records of the measurements derived from the EEG and those of the ICP in order to obtain a new multivariate series with the measurements derived from the EEG, both spectral and network, and the average ICP values in those intervals. This is an important step for integrating the underlying dynamics for both types of records. We have also used the electrocardiograph (ECG) record obtained by means of the V3 lead and from it we have calculated the heart rate by measuring the distance between T waves, meaning T-T. Although the EEG and ICP records are continuous, in some situations one or both records are cut off, such as when the patient is moved to other services in the hospital.

Finally, in order to quantify the dependence that some time series may have on others, we have used the Granger Causality (GC) [Granger, C. W. (1969). Investigating causal relations by econometric models and cross-spectral methods. Econometrica: Journal of the Econometric Society, 424-438.], estimated as follows: given two time series, each of them $Ndat$; X= $X_k$, $k$= 1; $Ndat$ and Y= $Y_K$, $K$= 1 $Ndat$, Granger causality basically examines whether the future values of one variable can be predicted by another. Numerically this can be evaluated by means of autoregressive models of order L that adjust each of the series such that:

$$x_k = \sum_{i=1}^{L} a_i x_{k-i} + \varepsilon_x$$

$$x_k = \sum_{i=1}^{L} a_i x_{k-i} + \varepsilon_x + \sum_{i=1}^{L} b_i y_{k-i} + \varepsilon_y$$

$$(1)$$

If the second prediction is *better* than the first one, it can be ensured that the past values of **y** affect the present values of **x**. The way to quantify "best" in a statistical sense is by means of a comparison between $\varepsilon_x$ and $\varepsilon_y$, for example, using the statistic:

$$F_{y \to x} = \ln \frac{\mathrm{var}(\varepsilon_x)}{\mathrm{var}(\varepsilon_y)}$$

$$(2)$$

Such that $F_{y \to x}$ is non-negative and the larger $F_{y \to x}$ is, the better the fit in the combined model and therefore this implies a causality of **y** over **x**. The statistical significance of this equation can be assessed by means of Fisher's test.

$$F_{y \to x} = \frac{\dfrac{RSS_x - RSS_y}{L}}{\dfrac{RSS_y}{N_{dat} - 2L - 1}}$$

$$(3)$$

Where *RSSx* and *RSSy* are the residual sums of the squares of the **x** and **y** models, respectively. In our case, of course, **x** and **y** will be replaced by the variables ICP (or ICP-diff) and the measurements from the EEG.

To confirm the relationship between the ICP and the electroencephalographic activity, we calculated different measurements of dependence and/or correlation between pairs of variables. Regarding the ICP we used this variable as well as the differential thereof, meaning the first derivative thereof, since in this manner any trend existing in the time series is removed, thus making it more stationary.

We used the GC to study a possible dependence of one time series on another. To do so, we studied the GC for all the pairs that can be formed between the following variables: Delta, Theta, Alpha, density of links, Spectral Entropy, ICP and the differential of the ICP. We have used Equation (1) for each of the time series in each pair of variables and we calculated the statistic determined by Equation (2). We have calculated the statistical significance of this estimator by means of Fisher's test, given by Equation (3). The continuous monitoring of the EEG and the ICP enables us to have records with an extensive duration and therefore enables us to study the dynamics of the interactions between the variables. In order to quantify this, we have divided the total record into 30-minute time windows. In each of these time windows we have calculated the GC for each pair of variables. Taking into account that the data, both from the ICP and the EEG measurements, is sampled every 5 seconds (see explanation in the methodology section), we will have to make the GC evaluations over a length of 30x12 = 360 values (12 values per minute) in 30 minutes.

As shown in Figure 2, there is a clear dependence on the ICP variable (and on ICP-diff, that is, the differential thereof), with respect to the measurements derived from the EEG, spectral entropy and the Delta, Theta and Alpha bands. It can also be seen that there is no such dependence in the opposite direction, or with respect to the network variable such as density of links (density). Although the dependence of the EEG variables on the ICP occurs throughout the entire record, in some areas it is more intense (for example, at about 11 PM on day 24) and less intense and even non-existent in other regions, such as for example at 9 AM on day 25.

Alternatively, we calculated the degree of correlation between all the pairs of variables in order to have an independent measurement of the degree of dependence existing between the variables studied. To do so, we calculated the cross-correlation between all the pairs of variables, in the same manner we used to study the GC. In order to study whether there is lead or lag in the correlation, which would imply causality, we have calculated the cross-correlation between all the pairs of variables with lag of up to a maximum of 30, meaning 30 displacements for one side and the other, between the two variables studied.

From the data shown in Figure 3, it is concluded that there is no correlation with lag (or lead) between variables derived from the EEG, but it does exist between the variables from the EEG and the ICP (and the differential thereof). In most cases, it is clear that there is lead (coloured blue) in the maximum value of the correlation, this fact implying that the variables derived from the EEG have lag with respect to the ICP. According to the colour scale of the graph, on average this lag is between 5 and 10 steps. Given that these steps are actually 5-second time windows, this would correspond to a lag of 25 and 50 seconds. Curiously, there is also a certain correlation between the density of links and the ICP, although there is no pattern that clearly identifies what variable has lead compared to which.

According to the previous results, the dependence between the measurements of the EEG and the ICP for 18 patients admitted to the ICU of the Hospital de la Princesa was checked. In these cases, we have found that in the majority of patients there was a strong dependence between the variables of the EEG (the bands and spectral entropy) on the ICP. In the same manner the calculations were made in Figure 2, we calculated the GC over the time in which the patients were monitored during their stay in the ICU, of both ICP and EEG. An example for one of the patients can be seen in table 1. We have calculated the "percentage" of time in which the GC is significant (P>0.05) during the entire monitoring process. This calculation has been made for different values of L, meaning the length of the autoregressive model in equation (1) which is somehow related to the lag existing between both variables.

Table 1: significant GC percentages for different variables of a patient.

|  | Lag=5 | Lag=10 | Lag=15 |
|---|---|---|---|
| Spectral entro -> icp | 60.87 | 57.39 | 49.57 |
| Spectral entro -> icp-diff | 60.87 | 57.39 | 50.43 |
| alpha -> icp | 52.17 | 46.09 | 43.48 |
| alpha -> icp-diff | 47.83 | 46.96 | 41.74 |
| delta -> icp | 57.39 | 53.91 | 49.57 |
| delta -> icp-diff | 53.91 | 53.91 | 46.09 |
| icp -> Spectral entro | 1.74 | 0.87 | 2.61 |
| icp-diff -> Spectral entro | 0.87 | 0.87 | 0.87 |
| icp -> alpha | 1.74 | 2.61 | 1.74 |
| icp-diff -> alpha | 0.00 | 1.74 | 0.00 |
| icp -> delta | 1.74 | 2.61 | 0.87 |
| icp-diff -> delta | 0.00 | 0.00 | 0.87 |

This same procedure has been performed in the set of 18 patients studied. Figure 4 shows the percentages of the GC throughout all the records for each of the patients, and for three lag values, Lag=5, 10 and 15.

The first impression of the figure is that, except in a few cases (patients 13 and 22), the trend observed in the example in table 1 is repeated, meaning that the percentages of GC between the spectral variables of the EEGs are much greater than the inverse ones, which in most cases disappear. Second, it is notable that the percentages vary in the same patient according to the lag existing between both time series. For example, in patient 11, the percentage of causality between the variables of the EEG over the ICP is much greater when the lag is 15, compared to a lag of 5 or 10. This fact would imply that the lag or duration of the causality effect of one variable over another is dependent on the patient.

Finally, an automatic classification method, Support Vector Machines (SVM), was used to determine a model that would enable the increase in ICP to be inferred. SVM is a non-linear classification algorithm [Chang C.C., Lin C.J. (2011). LIBSVM: A library for support vector machines. ACM Trans Intell Syst Technol TIST, 2:27.]. SVM was used as follows: all the time windows of all the patients were grouped in order to construct a general model, two-thirds of the randomly-chosen windows were used to train the algorithm, each window was assigned an ICP value calculated by the invasive method, in addition to all the variables derived from the EEG; one third of the randomly-chosen windows were used to determine the efficiency of the model. By means of a confusion matrix, the prediction percentage of the model was determined, which reached 91% prediction, decreasing to 89% when subtracting chance.

Our work analysing simultaneous EEG and ICP records in patients hospitalised in the ICU of the Hospital de la Princesa shows the existence of a direct relationship between the dynamics of the ICP and certain variables calculated from the EEG records.

The data was calculated from a device (7) comprising means for determining the values of the spectral analysis (8) and network (9) variables for an electroencephalogram, as well as means for calculating the transfer function (10) from said variables. Optionally, it contained a microprocessor (11), means for recording the electroencephalographic signal (12) and electrocardiographic signal (13), a supervised learning algorithm (14) and a screen to see the data (15).

**Claims**

1. A method for determining changes in intracranial pressure using data obtained from an electroencephalogram recorded for a patient comprising:

   a) determining the values of the spectral analysis and network variables for the electroencephalogram,
   b) determining the endogenous variable X for a transfer function the exogenous variables of which are the values for the variables obtained in step a),

   whereby changes in the endogenous variable X are indicative of changes in the value of the intracranial pressure.

2. The non-invasive method for determining intracranial pressure according to claim 1, wherein the spectral analysis variables for the electroencephalogram determined in step a) are selected from among: bands lower than delta (<1Hz), delta (1-4Hz), theta (4-7Hz), alpha (8-12Hz), beta (12-30Hz), gamma (30-100Hz), bands greater than gamma (>100Hz), spectral entropy of all frequencies.

3. The non-invasive method for determining intracranial pressure according to any of claims 1 to 2, wherein the network analysis variables for the electroencephalogram determined in step a) are selected from among the variables obtained from the synchronisation between the different electrodes of the electroencephalogram: density of links, average clustering coefficient, mean path length and degree of each node.

4. The non-invasive method for determining intracranial pressure according to any of claims 1 to 3, wherein the value of the spectral analysis and network variables of stage a) is determined by the following steps:

   I. calculating the power spectrum in the neurophysiological records from each electrode, by means of the Fourier transform;
   II. calculating the relative power of each of the characteristic frequency bands of the electroencephalogram;
   III. calculating the value of entropy and spectral entropy;
   IV. calculating synchronisation measurements between all pairs of neurophysiological records using Pearson correlation, Mutual Information and Phase Synchronisation;
   V. calculating the value of the density of links, average clustering coefficient, mean path length and degree of each node;
   VI. estimating all the previous measurements in consecutive time windows.

5. The non-invasive method according to any of claims 1 to 4, comprising determining variables associated with the heart rate variability of an electrocardiogram recorded for a patient.

6. The non-invasive method according to any of claims 1 to 5, comprising determining the variables associated with the sedation of a patient.

7. A device (7) for determining intracranial pressure in a patient by means of the method according to any of claims 1-6, comprising means for determining the values of the spectral analysis (8) and network (9) variables for an electroencephalogram; and means for calculating the transfer function (10) from said variables.

8. The device (7) according to claim 7, comprising a microprocessor (11).

9. The device (7) according to any of claims 7 to 8, comprising means for recording the electroencephalographic signal (12) and electrocardiographic signal (13).

10. The device (7) according to any of claims 7 to 9, comprising a supervised learning algorithm (14).

FIG.1

## FIG.2

FIG.3

Maximum correlation time

Alpha- Spectral Entropy
Theta- Spectral Entropy
Theta-Alpha
Delta- Spectral Entropy
Delta-Alpha
Delta-Theta
ICP-diff- Spectral Entropy
ICP-diff-Alpha
ICP-diff-Theta
ICP-diff-Delta
ICP- Spectral Entropy
ICP-Alpha
ICP-Theta
ICP-Delta
ICP-ICP-diff
density- Spectral Entropy
density-Alpha
density-Theta
density-Delta
density-ICP-diff.
density-ICP

Time (Y-M-D h:m:s)

FIG.4

FIG.5

# INTERNATIONAL SEARCH REPORT

| International application No. |
| --- |
| PCT/ES2018/070334 |

## A. CLASSIFICATION OF SUBJECT MATTER

See extra sheet

According to International Patent Classification (IPC) or to both national classification and IPC

## B. FIELDS SEARCHED

Minimum documentation searched (classification system followed by classification symbols)
A61B, G16H

Documentation searched other than minimum documentation to the extent that such documents are included in the fields searched

Electronic data base consulted during the international search (name of data base and, where practicable, search terms used)

EPODOC, INVENES, WPI, INTERNET

## C. DOCUMENTS CONSIDERED TO BE RELEVANT

| Category* | Citation of document, with indication, where appropriate, of the relevant passages | Relevant to claim No. |
| --- | --- | --- |
| A | CHEN, HUI, et al. . A new method of intracranial pressure monitoring by EEG power spectrum analysis. Canadian Journal of Neurological Sciences, 02/12/2014, Vol. 39, N° 4, Pages 483-487 [on line][retrieved on 26/06/2018]. Retrieved of Internet <URL: https://www.cambridge.org/core/services/aop-cambridge-core/content/view/94E48EBB0B33780C09ED53AF295E341A/S031716 7100013998a.pdf/new_method_of_intracranial_pressure_monitoring_by_eeg_power_spectrum_analysis.pdf >, <DOI: 10.1017/S0317167100013998> (abstract); paragraphs 1, 3, discussion | 1-10 |

☒ Further documents are listed in the continuation of Box C.　　　☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | "&" document member of the same patent family |

| Date of the actual completion of the international search | Date of mailing of the international search report |
| --- | --- |
| 31/08/2018 | **(05/09/2018)** |

| Name and mailing address of the ISA/ | Authorized officer |
| --- | --- |
| OFICINA ESPAÑOLA DE PATENTES Y MARCAS Paseo de la Castellana, 75 - 28071 Madrid (España) Facsimile No.: 91 349 53 04 | J. Vazquez Burgos Telephone No. 91 3495513 |

Form PCT/ISA/210 (second sheet) (January 2015)

**INTERNATIONAL SEARCH REPORT**

International application No.

PCT/ES2018/070334

C (continuation).                    DOCUMENTS CONSIDERED TO BE RELEVANT

| Category * | Citation of documents, with indication, where appropriate, of the relevant passages | Relevant to claim No. |
|---|---|---|
| A | POPOVIC, DJORDJE; KHOO, MICHAEL; LEE, STEFAN. Noninvasive monitoring of intracranial pressure. Recent patents on biomedical engineering, 01/11/2009, Vol. 2, N° 3, Pages 165-179 [on line][retrieved the 27/06/2018]. Retrieved from Internet <URL:https://www.researchgate.net/profile/Djordje_Popovic/publication/228785261_Noninvasive_Monitoring_of_Intracranial_Pressure/links/553687550cf268fd00186ee6.pdf > paragraphs 5.1, 6 | 1-10 |
| L | PAHREN, L. A. A Case Study on Neurophysiologic Data from Patients with Traumatic Brain Injury. Tesis Doctoral. 31/12/2007 [on line][retrieved the 21/06/2018]. Retrieved from Internet <URL:https://etd.ohiolink.edu/pg_10?0::NO:10:P10_ACCESSION_NUM:ucin1490352193060328#abstract-files > paragraphs 1.1-1.2, 2.1-2.3, 3.1-3.3, 3.5, 3.7, 5 | 1-10 |
| A | BULLMORE, ED; SPORNS, OLAF. Complex brain networks: graph theoretical analysis of structural and functional systems. Nature Reviews Neuroscience, 04/02/2009, Vol. 10, N° 3, Pages 186-198 [on line][retrieved on 21/06/2018]. Retrieved of Internet <URL: https://www.nature.com/articles/nrn2575 > pages 186 - 194; | 1-10 |

Form PCT/ISA/210 (continuation of second sheet) (January 2015)

## INTERNATIONAL SEARCH REPORT

International application No.

PCT/ES2018/070334

### CLASSIFICATION OF SUBJECT MATTER

*A61B5/03* (2006.01)
*A61B5/0476* (2006.01)
*G16H10/60* (2018.01)
*G16H50/20* (2018.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LE ROUX, P. ; MENON, D. K. ; CITERIO, G. ; VESPA, P. ; BADER, M. K. ; BROPHY, G. M. ; BAD-JATIA, N.** Consensus summary statement of the international multidisciplinary consensus conference on multimodality monitoring in neurocritical care. *Neurocritical care,* 2014, vol. 21 (2), 1-26
- **SANZ-GARCÍA, A. ; VEGA-ZELAYA, L. ; PASTOR, J. ; SOLA, R. G. ; ORTEGA, G. J.** Towards Operational Definition of Postictal Stage: Spectral Entropy as a Marker of Seizure Ending. *Entropy,* 2017, vol. 19 (2), 81
- **MEZEIOVA K. ; PALUU M.** Comparison of coherence and phase synchronization of the human sleep electroencephalogram. *Clin Neurophysiol,* 2012, vol. 123, 1821-1830
- **KIRKMAN, M. A. ; SMITH, M.** Intracranial pressure monitoring, cerebral perfusion pressure estimation, and ICP/CPP-guided therapy: a standard of care or optional extra after brain injury?. *British journal of anaesthesia,* 2013, aet418
- **KRISTIANSSON, H. ; NISSBORG, E. ; BARTEK, J. ; ANDRESEN, M. ; REINSTRUP, P. ; ROMNER, B.** Measuring Elevated Intracranial Pressure through Noninvasive Methods: A Review of the Literature. *J Neurosurg Anesthesiol,* 2013, vol. 25 (4), 372-85
- **MARTÍNEZ-MAÑAS, R. M. ; SANTAMARTA, D. ; DE CAMPOS, J. M. ; FERRER, E.** Camino® intracranial pressure monitor: prospective study of accuracy and complications. *Journal of Neurology, Neurosurgery & Psychiatry,* 2000, vol. 69 (1), 82-86
- **GRANGER, C. W.** Investigating causal relations by econometric models and cross-spectral methods. *Econometrica: Journal of the Econometric Society,* 1969, 424-438
- **CHANG C.C. ; LIN C.J.** LIBSVM: A library for support vector machines. *ACM Trans Intell Syst Technol TIST,* 2011, vol. 2, 27